(19) Europäisches Patentamt
European Patent Office
Office européen des brevets

(11) Veröffentlichungsnummer: **0 409 227 A2**

# (12) EUROPÄISCHE PATENTANMELDUNG

(21) Anmeldenummer: **90113851.1**

(51) Int. Cl.[5]: **C07H 19/04**, A61K 31/70

(22) Anmeldetag: **19.07.90**

(30) Priorität: **20.07.89 DD 331051**

(43) Veröffentlichungstag der Anmeldung:
**23.01.91 Patentblatt 91/04**

(84) Benannte Vertragsstaaten:
**AT BE CH DE DK ES FR GB GR IT LI LU NL SE**

(71) Anmelder: **Akademie der Wissenschaften der DDR**
**Otto-Nuschke-Strasse 22/23**
**DDR-1086 Berlin(DD)**

(72) Erfinder: **Matthes, Eckart, Dr.**
**Karower Chaussee 129**
**DDR-1115 Berlin(DE)**
Erfinder: **Von Janta-Lipinski, Martin, Dr.**
**Mittelweg 75**
**DDR-1185 Berlin(DE)**
Erfinder: **Reimer, Karen, Dr.**
**Leipziger Strasse 40**
**DDR-1080 Berlin(DE)**
Erfinder: **Müller, Werner, Prof. Dr.**
**Semmelweisstrasse 12**
**D-6200 Wiesbaden(DE)**
Erfinder: **Meisel, Helga, Dr.**
**Strasse 53 Nr. 5a**
**DDR-1113 Berlin(DE)**
Erfinder: **Lehmann, Christine**
**Walter-Friedrich-Strasse 5**
**DDR-1115 Berlin(DE)**
Erfinder: **Schildt, Jürgen**
**Demminer Strasse 11c**
**DDR-1090 Berlin(DE)**

(74) Vertreter: **Patentanwälte Beetz sen. - Beetz jun. Timpe - Siegfried - Schmitt-Fumian-Mayr**
**Steinsdorfstrasse 10**
**D-8000 München 22(DE)**

(54) **Pyrimidin - und Purinnucleoside, ihre Herstellung und ihre pharmazeutische Verwendung.**

(57) Die Erfindung betrifft neue und bekannte Pyrimidin- und Purinnucleoside, die besonders wirksam gegen Hepatitis B-Infektionen sind und ihre Herstellung.

EP 0 409 227 A2

## PYRIMIDIN- UND PURINNUCLEOSIDE, IHRE HERSTELLUNG UND IHRE PHARMAZEUTISCHE VERWENDUNG

Die Erfindung betrifft Pyrimidin- und Purinnucleoside, Verfahren zu ihrer Herstellung und ihre Verwendung als pharmazeutische Wirkstoffe bzw. Mittel zur Prophylaxe und/oder Behandlung von durch Hepatitis B-Viren verursachten Infektionen, insbesondere in der Humanmedizin.

Die Hepatitis B ist eine durch das Hepatitis B- virus (HBV) verursachte Infektionskrankheit, von der weltweit etwa 200 Millionen Menschen betroffen sind und deren chronische Form mit einem erhöhten Risiko für ein primäres Leber-Carcinom verbunden ist, das allein in China zu etwa einer Million Tumorneuerkrankungen pro Jahr führt.

Eine wirksame und verträgliche antivirale Therapie fehlt bisher. Angriffspunkt hönnte u.a. die HBV-DNA-Polymerase sein, ein Enzym, dessen Hemmung die weitere intracelluläre Virusvermehrung verhindern und damit seine Ausbreitung stoppen könnte. Die ersten klinisch erprobten Hemmstoffe der HBV-Polymerase, wie z.B. Adeninarabinosid(araA) und Acyclovir (ACV), haben auch in Kombination mit Corticosteroiden oder Interferon-α nur teilweise oder vorübergehende Besserungen erbracht oder sind von Nebenwirkungen begleitet (Alexander et al., British Medical Journal 292, 915 (1986), so daß die Notwendigkeit besteht, wirksamere und selektivere Arzneimittel zu entwickeln. In diese Richtung potentiell wirksamer Hemmstoffe weisen die Patentanmeldungen WO-A-88/00050, in der 3′-Fluordesoxynucleoside von Adenin, Guanin, Cytosin und Thymin als Mittel gegen Hepatitis B-Infektionen beansprucht werden, WO-A-89/01776 bezüglich der Wirksamkeit von 2′-Fluorarabinofuranosyl-5-ethyluracil gegenüber der Woodchuck-Hepatitis und EP-A-303 760, das verschiedene Purin-2′,3′-didesoxynucleoside als effektive Hemmstoffe des Entenhepatitis B-Virus beschreibt.

Einige dieser Nucleoside wurden ursprünglich als Hemmstoffe der Replikation des HIV (human immunodeficiency virus, eines RNA-Virus) entdeckt, ohne daß aus der Wirksamkeit von Nucleosiden gegenüber HIV auf ihre prinzipielle Wirksamkeit gegenüber HBV (einem DNA-Virus) geschlossen werden kann, wie das im Falle von EP-A-322 384 erfolgt ist.

Die Erfindung hat die Aufgabe, neue Pyrimidin- und Purinnucleoside und ihre Herstellung anzugeben, die gegen Hepatitis B wirksam sind und als pharmazeutische Mittel für die Prophylaxe und/oder Behandlung entsprechender Infektionen eingesetzt werden können, und ferner, weitere wirksame Verbindungen gegen Hepatitis B aufzuzeigen, die bei hoher Wirksamkeit und guter Verträglichkeit eine geringe Toxizität aufweisen.

Die Aufgabe wird gemäß den Ansprüchen 1, 7, 8 und 9 gelöst. Vorteilhafte Weiterbildungen sind Gegenstand der abhängigen Ansprüche.

Die erfindungsgemäßen neuen Verbindungen besitzen die allgemeinen Formeln I, II, III, IV und V,

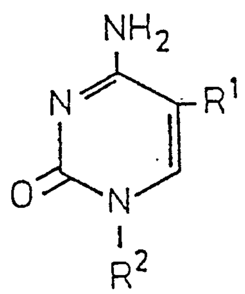

(I),

worin bedeuten: $R^1$ CHO, $NH_2$, OH, SH
und

$R^2$

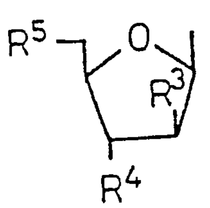

mit $R^3$ = H, OH

$R^4$ = H, F, Cl, $NH_2$, $N_3$
$R^5$ = OH, O-Acetyl, O-Palmitoyl, O-Alkoxycarbonyl, Phosphonat, Mono-, Di-, Triphosphat bzw. andere Precursorgruppen für die 5-Hydroxygruppe,

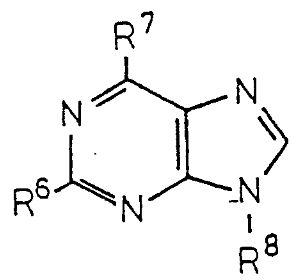

(II),

worin bedeuten:
$R^6$ H, OH, F, Cl, Br, $NH_2$, SH, N-Dialkyl, insbesondere bis $C_3$,
$R^7$ H, OH, F, Cl, Br, $NH_2$, SH, N-Dialkyl, insbesondere bis $C_3$,
$R^8$ 2,3-Didesoxy-3-aminoribofuranosyl, 2,3-Didesoxy-3-chlorribofuranosyl, 2,3-Didesoxy-3-azidoribofuranosyl, 2,3-Didesoxy-3-fluorribofuranosyl, Arabinofuranosyl 3-Fluorarabinofuranosyl, 2,3-Didehydro-2,3-didesoxyribofuranosyl bzw. deren 5-Phosphonat 5-O-Acetyl-, 5-O-Palmitoyl-, 5-O-alkoxycarbonyl-Derivate oder deren 5-Mono-, 5-Di-, oder 5-Triphosphate (als freie Säuren oder Alkali-, Ammonium oder Alkylammonium salze) bzw. andere Precursorgruppen für die 5-Hydroxygruppe, mit folgenden Einschränkungen:

Wenn $R^6$ und $R^7$ = H, OH, $NH_2$,
dann ist
$R^8 \neq$ 2,3-Didesoxy-3-chlorribofuranosyl, 2,3-Didesoxy-3-aminoribofuranosyl, 2,3-Didesoxy-3-azidoribofuranosyl, 2,3-Didesoxy-3-fluorribofuranosyl, Arabinofuranosyl 3-Fluorarabinofuranosyl, 2,3-Didehydro-2,3-didesoxyribofuranosyl;
oder wenn $R^6$ = F und $R^7$ = $NH_2$ bzw. $R^6$ = $NH_2$ und $R^7$ = SH, dann ist $R^8 \neq$3-Fluorarabinofuranosyl bzw. 2,3-Didesoxy-3-fluorribofuranosyl,
oder wenn $R^6$ = Cl und $R^7$ = $NH_2$, dann ist $R^8 \neq$ 2,3-Didehydro-2,3-didesoxyribofuranosyl;

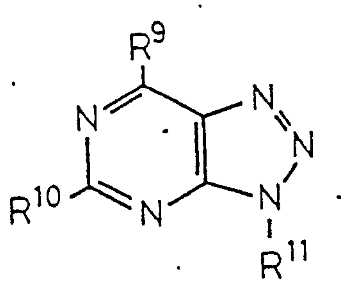

(III),

worin bedeuten:
$R^9$ und $R^{10}$ H, OH, $NH_2$ und
$R^{11}$ 2,3-Didehydro-2,3-didesoxyribofuranosyl oder

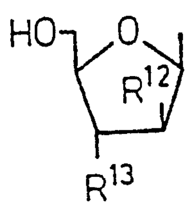

mit $R^{12}$ = H, OH
$R^{13}$ = H, F, Cl, $NH_2$, $N_3$
bzw. mit einer Precursorgruppe für die 5-Hydroxygruppe wie bei $R^8$;

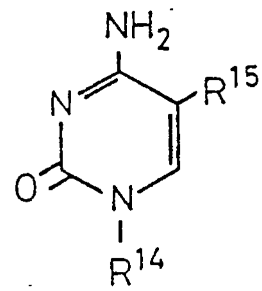

(IV),

worin bedeuten:

$R^{14}$ 2,3-Didehydro-2,3-didesoxyribofuranosyl bzw. eine Precursorgruppe für die 5-Hydroxygruppe wie bei $R^8$ und

$R^{15}$ F, Br, J, $CH_3$, $CH_2OH$, $CH_2CH_3$, CHO;

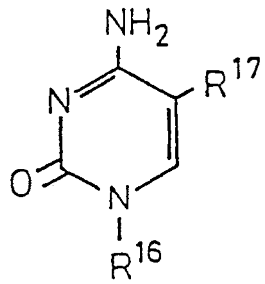

(V),

worin bedeuten:

$R^{16}$ Arabinofuranosyl, 2,3-Didesoxy-3-aminoribofuranosyl bzw. mit einer Precursorgruppe für die 5-Hydroxygruppe wie bei $R^8$, und

$R^{17}$ Cl, Br, $CH_2OH$, $CH_2CH_3$, $CH=CH_2$, $CH=CHBr$.

Diese Pyrimidin- und Purinnucleoside stellen Wirkstoffe gegen Virusinfektionen dar, insbesondere gegen Infektionen, wie sie durch das Hepatitis B-Virus (HBV), das Hepatitis A-Virus (HAV) und den Erreger der Non A-, Non B-Hepatitis verursacht werden. Sie können als solche sowie in Form pharmazeutischer Mittel zusammen mit geigneten Hilfs- und/oder Trägerstoffen zur Behandlung und/oder Prophylaxe von Hepatitis B vorteilhaft eingesetzt werden, insbesondere in der Humanmedizin.

Als besonders wirksam erwiesen sich:

2′,3′-Didesoxy-3′-fluor-5-formylcytidin (I),
2′,3′-Didesoxy-3′-fluorribofuranosyl-6-chlorguanin (II),
2′,3′-Didesoxy-3′-fluorribofuranosyl-8-azaguanin (III),
2′,3′-Didehydro-2′,3′-didesoxy-5-methylcytidin (IV),
Arabinofuranosyl-5-hydroxymethylcytosin (V).

Sie sind gegenüber den Verbindungen des Standes der Technik gut wirksam und weisen weniger Toxizität und Nebenwirkungen auf. Ihre Herstellung erfolgt nach an sich bekannten Verfahren.

Zur Herstellung der Verbindungen I werden entweder 3′-modifizierte 5-Methylpyrimidinnucleoside zunächst bromiert, und anschließend wird das exocyclische Brom eliminiert, oder das entsprechende 5-Bromderivat wird substituiert.

Die Verbindungen II und III werden aus den entsprechenden Purinen in einer Transglycosidierungsreaktion mit dem entsprechenden Zucker hergestellt, oder das entsprechende 3′-Tosylderivat wird einer Eliminierungsreaktion unterzogen.

Die Verbindungen IV erhält man aus den 3′,5′-Di-O-mesylverbindungen, die am $N^4$ geschützt sind, durch Eliminierung.

Zur Herstellung der Verbindungen V werden das entsprechende Arabinofuranosylcytosin bzw. das 2′,3′-Didesoxy-3′-aminocytidin in der 5-Position durch Halogen substituiert, oder das 5-Ethylarabinofuranosylcytosin bzw. das 2′,3′-Didesoxy-3′-amino-5-ethylcytidin wird durch Bromierung und nachfolgende Eliminierung abgewandelt.

Eine Behandlung und/oder Prophylaxe von Hepatitis-Infektionen in der Humanmedizin besteht gemäß der Erfindung in der Verabreichung einer wirksamen Menge einer oder mehrerer Verbindungen der Formeln I, II, III, IV und/oder V als solche oder in Form geeigneter Formulierungen.

Die erfindungsgemäßen pharmazeutischen Mittel, die insbesondere zur Prophylaxe und/oder Therapie von Hepatitis-Infektionen in der Humanmedizin geeignet sind, enthalten entsprechend mindestens ein Pyrimidin- bzw. Purinnucleosid der Formeln I, II, III, IV und/oder V, wie oben definiert, als Wirkstoff neben einem oder mehreren pharmazeutisch geeigneten Hilfs- und/oder Trägerstoffen und gegebenenfalls anderen therapeutischen Mitteln oder Wirkstoffen. Die Mittel werden vorteilhaft in Form von Dosiereinheiten mit einer oder mehreren Einheitsdosen hergestellt.

Die Erfindung betrifft ferner die Verwendung von Verbindungen der allgemeinen Formeln VI und VII,

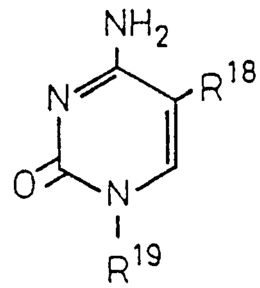

(VI),

in der bedeuten:
$R^{18}$ F, Cl, Br, J, $CH_3$, $CH_2OH$, $C_2H_5$ und
$R^{19}$ 2,3-Didesoxyribofuranosyl, 2,3-Didesoxy-3-azidoribofuranosyl, 2,3-Didesoxy-3-fluorribofuranosyl, 2,3-Didesoxy-3-chlorribofuranosyl, 3-Fluorarabinofuranosyl bzw. eine Precursorgruppe für die 5 Hydroxygruppe wie bei $R^8$
oder
$R^{18}$ F, J, $CH_3$ und
$R^{19}$ Arabinofuranosyl, 2,3-Didesoxy-3-aminofuranosyl bzw. eine Precursorgruppe für die 5 Hydroxygruppe wie bei $R^8$
oder
$R^{18}$ H, Cl und
$R^{19}$ 2,3-Didehydro-2,3-didesoxyribofuranosyl bzw. eine Precursorgruppe für die 5 Hydroxygruppe wie bei $R^8$

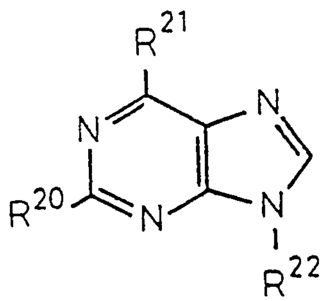

(VII),

in der bedeuten:
$R^{20}$ $NH_2$ und $R^{21}$ SH,
$R^{20}$ $NH_2$ und $R^{21}$ H
$R^{20}$ $NH_2$ und $R^{21}$ $NH_2$
$R^{20}$ F und $R^{21}$ $NH_2$, und
$R^{22}$ 2,3-Didesoxy-3-fluorribofuranosyl, 3-Fluorarabinofuranosyl bzw. eine Precursorgruppe für die 5 Hydroxygruppe wie bei $R^8$
oder
$R^{20}$ H und $R^{21}$ $NH_2$
$R^{20}$ $NH_2$ und $R^{21}$ OH
$R^{20}$ $NH_2$ und $R^{21}$ $NH_2$, und
$R^{22}$ 2,3-Didehydro-2,3-didesoxyribofuranosyl 2,3-Didesoxy-3-aminoribofuranosyl bzw. eine Precursorgruppe für die 5 Hydroxygruppe wie bei $R^8$
oder

$R^{20}$ CH und $R^{21}$ $NH_2$, und

$R^{22}$ 2,3-Didesoxy-3-fluorribofuranosyl bzw. eine Precursorgruppe für die 5 Hydroxygruppe wie bei $R^8$,

zur Herstellung pharmazeutischer Mittel

zur Prophylaxe und/oder Behandlung von durch Hepatitis B-Viren verursachten Infektionen, insbesondere in der Humanmedizin.

Von besonderer Bedeutung sind dabei:

a) Arabinofuranosyl-5-methylcytosin,

b) 2′,3′-Didesoxy-3′-fluor-5-methylcytidin-5′-hydrogenphosphonat,

c) 2′,3′-Didesoxy-3′-fluor-5-methylcytidin,

d) 2′,3′-Didesoxy-3′-azido-5-methylcytidin,

e) 2′,3′-Didesoxy-3′-aminoribofuranosylguanin,

f) 2′,3′-Didesoxy-3′-fluorarbinofuranosyl-5-methylcytosin,

g) 2′,3′-Didehydro-2′,3′-didesoxyribofuranosylguanin und

h) 2′,3′-Didesoxy-3′-fluorribofuranosyl-2,6-diaminopurin.

Die Herstellung der Verbindungen c), f) und h) ist in EP-A-305 114 beschrieben.

Die Verbindungen der Formeln VI und VII stellen besonders wirksame Verbindungen zur Behandlung und/oder Prophylaxe von Hepatitis B-Infektionen dar.

Sie werden eingesetzt zur Herstellung entsprechender pharmazeutischer Mittel.

Die pharmazeutischen Mittel werden in Form von Dosiereinheiten oder Vielfachen davon hergestellt. Die eingesetzten Hilfs- und Trägerstoffe müssen verträglich, mit den anderen Bestandteilen der Mittel kompatibel und für den Patienten unschädlich sein.

Zu den erfindungsgemäßen galenischen Formulierungen bzw. Applikationsformen der pharmazeutischen Mittel gehören solche, die für die orale, rektale, nasale, topische, vaginale oder parenterale (einschließlich subkutaner, intramuskulärer, intravenöser und intradermaler) Verabreichung geeignet sind. Ein bzw. mehrere Wirkstoffe werden mit dem Träger, der aus mehreren Begleitbestandteilen zusammengesetzt sein kann, in Kontakt gebracht und, falls erforderlich, in eine entsprechende galenische Form übergeführt.

Die pharmazeutischen Mittel für orale Verabreichung werden in Form von Dragees, Tabletten, Kapseln, als Pulver oder als Granulate hergestellt, die jeweils eine bestimmte Menge des Wirkstoffs enthalten. Ebenso können sie als Lösungen oder als Suspensionen formuliert werden. Gegebenenfalls werden Geschmacksmittel und/oder andere übliche Additive zugesetzt.

Mittel für die rektale Verabreichung werden als Zäpfchen mit einer geeigneten Grundlage hergestellt. Arzneimittel für eine vaginale Verabreichung werden z.B. als Pessare, Tampons, Cremen, Gele, Pasten, Schäume oder Spray-Produkte formuliert.

Die Mittel für die parenterale Verabreichung können eine Dosiereinheit des Wirkstoffs oder das Mehrfache davon enthalten und beispielsweise in Ampullen, Phiolen oder in einem gefriergetrockneten Zustand lagerfähig gemacht sein. Unmittelbar vor Gebrauch hergestellte Injektionslösungen und Suspensionen können aus sterilen Pulvern, Granulaten und Tabletten erhalten werden, z.B. durch Lösen der Substanz in physiologischer Kochsalzlösung, Glucose oder anderen für die I.v.-Injektion bzw. Infusion geeigneten Medien.

Zur Behandlung von Patienten mit Heptitis B-Infektionen wird beispielsweise aus 2′,3′-Didesoxy-3′-fluor-5-methylcytidin und physiologischer Kochsalzlösung die erforderliche Menge einer 0,3%igen Lösung hergestellt, die sterilisiert und dem Patienten innerhalb einer Stunde intravenös infundiert wird. Die Infusion wird über mindestens 8 bis 10 Tage alle 4 bis 8 Stunden wiederholt.

Die Wirksamkeit einiger der erfindungsgemäßen Verbindungen wird wie folgt belegt:

a) Hemmung der Hepatitis B Virus-Polymerase durch Nucleosidtriphosphat-Analoga

Das Hepatitis B Virus (HBV) besitzt eine zirkuläre, partiell doppelsträngige DNA, welche nach Infektion einer Leberzelle durch eine viruseigene Polymerase repliziert wird. Dieser Vorgang setzt jedoch die Synthese einer RNA durch eine zelluläre RNA-Polymerase voraus. Nur diese RNA-Matrize - im Zusammenhang mit der Replikation auch als Prägenom bezeichnet - kann von der HBV-Polymerase für die Synthese zunächst eines RNA-DNA Hybrids und dann der doppelsträngigen DNA benutzt werden. Damit verfügt die HBV-Polymerase über eine enzymatische Aktivität, die der einer reversen Transcriptase ähnelt (Will et al., J.Virology 61, 904 (1987)). Es wurde zunächst die Hemmbarkeit der HBV-assoziierten Polymerase durch die 5′-Triphosphate der erfindungsgemäßen Verbindungen untersucht. Dazu wurde die von Kaplan et al. (J.Virology 12, 995 (1973)) angegebene Methode zur Bestimmung der HBV-Polymerase benutzt, wobei die

zu testenden Triphosphate den Ansätzen in verschiedenen Konzentrationen zugesetzt wurden. Die Hemmung der Enzymreaktion wurde nach 3-stündiger Inkubation bei 37 °C durch Vergleich mit einem Kontrollwert ermittelt und daraus die Konzentration der zugesetzten Analoga bestimmt, die zu einer 50 %igen Hemmung der HBV-Polymerase-Aktivität führt ($ID_{50}$). Tabelle 1 zeigt die Ergebnisse, wobei insbesondere 2′,3′-Didesoxy-3′-fluor-5-methylcytidin durch einen Wert $ID_{50}$ von 0,03 µM herausragt und damit zu den stärksten bisher bekannten Hemmstoffen der HBV-Polymerase zu rechnen ist.

Tabelle 1

| Hemmung der HBV-Polymerase-Aktivität durch Nucleosidtriphosphat-Analoga. | | |
|---|---|---|
| Hemmstoff | Abkürzung | $ID_{50}$ (µM) |
| 2′,3′-Didesoxy-3′-fluor-5-methylcytidin-5′-triphosphat | FMetdCTP | 0,03 |
| 2′,3′-Didesoxy-3′-fluor-5-ethylcytidin-5′-triphosphat | FEtdCTP | 0,35 |
| 2′,3′-Didehydro-2′,3′-didesoxy-5-methylcytidin-5′-triphosphat | ddeMetCTP | 0,28 |
| 2′,3′-Didesoxy-5-methylcytidin-5′-triphosphat | ddMetCTP | 8 |
| 2′,3′-Didesoxy-3′chlor-5-methylcytidin-5′-triphosphat | ClMetdCTP | 9 |
| 2′,3′-Didesoxy-3′-azido-5-methylcytidin-5′-triphosphat | $N_3$MetdCTP | 0,12 |
| 2′,3′-Didesoxy-3′-amino-5-methylcytidin-5′-triphosphat | $NH_2$MetdCTP | 26 |
| 2′,3′-Didehydro-2′,3′-didesoxycytidin-5′-triphosphat | ddeCTP | 2,5 |
| 2′,3′-Didehydro-2′,3′-didesoxyadenosin-5′-triphosphat | ddeATP | 1,8 |
| 2′,3′-Didehydro-2′,3′-didesoxyguanosin-5′-triphosphat | ddeGTP | 1,6 |
| 2′,3′-Didesoxy-3′-fluorguanosin-5′-triphosphat | FdGTP | 0,15 |
| 2′,3′-Didesoxy-3′-fluor-6-thioguanosin-5′-triphosphat | | 0,5 |
| 2′,3′-Didesoxy-3′-fluor-2-aminopurinribosid-5′-triphosphat | | 0,6 |
| 2′,3′-Didesoxy-3′-aminoguanosin-5′-triphosphat | | 4,0 |

b) Wirkung der modifizierten Nucleosidtriphosphate auf die zellulären DNA-Polymerasen-alpha und -beta

Um zu prüfen, inwieweit die beschriebene Wirkung auf die HBV-Polymerase selektiv ist, d.h., die zellulären DNA-Polymerasen von einer Hemmung durch die erfindungsgemäßen Nucleosidtriphosphate ausgenommen sind, wurden die zellulären DNA-Polymerasen-alpha und -beta in die Untersuchungen einbezogen. Die Isolierung der Enzyme und deren Aktivitätsbestimmung erfolgte entsprechend der Methode von Matthes et al. (Biomed.Biochim.Acta 44, K63-K73 (1985)). Die Ergebnisse der Tabelle 2 zeigen, daß die für die Replikation der zellulären DNA zuständige DNA-Polymerase alpha weitgehend unbeeinflußt bleibt. Eine 50 %ige Hemmung dieses Enzyms wird mit Ausnahme auch bei Hemmstoffkonzentrationen >100 µM noch nicht erreicht, so daß die DNA-Polymerase-alpha diesen Verbindungen gegenüber als nahezu resistent anzusehen ist.

Für die DNA-Polymerase-beta, die als Reparaturenzym zellulärer DNA-Schäden angesehen wird, liegen die $ID_{50}$- Werte zwischen 1 und >200 µM. Damit besitzt die beschriebene Verbindung eine hohe Wirkungsspezifität.

Tabelle 2

| Wirkung der modifizierten Nucleosidtriphosphate auf die zellulären DNA-Polymerasen-alpha und -beta ($ID_{50}$) | | |
|---|---|---|
| Hemmstoff* | $ID_{50}$ ($\mu M$) DNA-Polymerase- | |
| | alpha | beta |
| FMetdCTP | > 200 | 1 |
| FEtdCTP | > 200 | 3 |
| ddeMetCTP | > 200 | 10 |
| ddMetCTP | > 200 | > 100 |
| ClMetdCTP | > 200 | 100 |
| $N_3$MetdCTP | > 200 | > 200 |
| $NH_2$MetdCTP | > 200 | > 200 |
| ddeCTP | > 200 | 6 |
| ddeATP | > 100 | 4,5 |
| ddeGTP | > 150 | 5 |
| FdGTP | > 200 | 1,8 |

* Abkürzungen siehe Tabelle 1

c) Cytotoxizität der modifizierten Nucleoside

Unter dem Aspekt einer möglichen Anwendung der Verbindungen am Menschen wurden ihre antiproliferativen Wirkungen an menschlichen Zellkulturen wie früher beschrieben getestet (E. Matthes et al. Biochem.Biophys.Res.Commun. 153, 825 (1988)). Dabei zeigte sich, daß z.B. 2′,3′-Didesoxy-3-fluor-5-methylcytidin gegenüber der T-Zellinie MT4 eine $CD_{50}$ von 190 $\mu M$ aufweist. In einem weiteren Test, in dem eine mögliche Wachstumshemmung koloniebildender Zellen des Knochenmarks der Maus (CFU-GM) untersucht wurde (E. Matthes et al. Biochem.Biophys.Res.Commun. 165, 488 (1989), erwies sich diese Verbindung ebenfalls als kaum proliferationshemmend ($CD_{50}$ > 500 $\mu M$). Die Wirkung weiterer Verbindungen auf die Proliferation von MT4-Zellen ist in Tabelle 3 zusammengefaßt.

Tabelle 3

| Wirkung einiger erfindungsgemäßer Nucleosid-Analoga auf die Proliferation von MT4 Zellen. Angegeben ist die Konzentration , die nach 48 Std. zu einer 50 %igen Hemmung der Zellvermehrung führt ($CD_{50}$) | |
|---|---|
| Hemmstoff | $CD_{50}$ (µM) |
| 2′,3′-Didesoxy-3′-fluor-5-methylcytidin | 190 |
| 2′,3′-Didesoxy-3′chlor-5-methylcytidin | 625 |
| 2′,3′-Didesoxy-3′-amino-5-methylcytidin | 1,2 |
| 2′,3′-Didesoxy-3′-azido-5-methylcytidin | > 500 |
| 2′,3′-Didesoxy-5-methylcytidin | 110 |
| 2′,3′-Didehydro-2′,3′-didesoxy-5-methylcytidin | 205 |
| 2′,3′-Didesoxy-3′-fluorguanosin | 250 |
| 2′,3′-Didehydro-2′,3′-didesoxyadenosin | 48 |
| 2′,3′-Didehydro-2′,3′-didesoxyguanosin | 155 |
| 2′,3′-Didesoxy-3′-aminoguanosin | 210 |

d) Hemmung der HBsAg-Sekretion von Hep G2 (2.2.15)-Zellen

Die Testung der antiviralen Wirksamkeit der Nucleoside auf zelluärer Ebene erfolgte an einer menschlichen Hepatoblastom-Zellinie, die mit dem HBV-Genom tranfiziert wurde und sowohl das Oberflächenantigen des Virus (HBsAg) als auch die HBV-DNA permanent produziert und infektiöse Viruspartikel in das Medium abgibt (Hep G2-Zellen, Klon 2.2.15, Sells et al. Proc.Natl.Acad. Sci. 84, 1005 (1987)). Die Zellen ($5x10^5$/ml) wurden in RPMI 1640-Medium mit 2 mM Glutamin und 10% fetalem Kälberserum allein oder zusammen mit den Nucleosid-Analoga für 4 Tage inkubiert, danach wurden den Ansätzen für 24 Stunden $^3$H-Thymidin bzw. $^3$H-Desoxyadenosin (3 µCi/ml) zugesetzt, die Zellen abzentrifugiert und darin die säureunlösliche Radioaktivität und damit der Einfluß auf die DNA-Synthese bestimmt. Im Inkubationsmedium wurde das HBsAg durch Festphasenradioimmunoassay (Travenol-Genentech Diagnostics, Cambridge, MA) entsprechend den Instruktionen des Herstellers ermittelt. Die Tabelle 4 faßt die Ergebnisse zusammen, die belegen, daß die getesteten Verbindungen hochwirksame und selektive Hemmstoffe der HBV-Vermehrung sind.

Tabelle 4 Einfluß einiger erfindungsgemäßer Nucleosid-Analoga auf die HBsAg-Sekretion von Hep G2 (2.2.15)-Zellen

Die Mittelwerte von fünf unabhängigen Versuchen mit den Standardabweichungen sind wiedergegeben.

| Nucleosid | Konz. (µM) | HBsAg (ng/ml) | HBsAg (%) | $^3$H-DNA (dpm/Kultur) |
|---|---|---|---|---|
| ohne | - | 19,3 ± 2,9 | 100 | 14310 ± 1785 |
| 2',3'-Didesoxy-3'-fluor-5-methylcytidin | 0,003 | 14,3 ± 2,4 | 74 | 13270 ± 1650 |
| | 0,01 | 7,0 ± 0,9 | 36 | 15060 ± 1895 |
| | 0,03 | 2,4 ± 0,3 | 12 | 15580 ± 1910 |
| | 0,1 | 0,4 ± 0,1 | 2 | 14350 ± 1520 |
| | 0,3 | < 0,1 | < 1 | 13175 ± 1975 |
| | 1,0 | < 0,1 | < 1 | 8295 ± 1010 |
| | 3,0 | < 0,1 | < 1 | 2470 ± 295 |
| 2',3'-Didesoxy-3'-fluor-5-ethylcytidin | 0,003 | 16,7 ± 2,6 | 87 | 14020 ± 1780 |
| | 0,01 | 8,2 ± 0,8 | 42 | 13855 ± 1945 |
| | 0,03 | 2,6 ± 0,6 | 13 | 14570 ± 1695 |
| | 0,1 | 0,6 ± 0,1 | 3 | 15245 ± 1655 |
| | 0,3 | < 0,1 | < 1 | 14130 ± 1940 |
| | 1,0 | < 0,1 | < 1 | 9850 ± 1230 |
| | 3,0 | < 0,1 | < 1 | 6640 ± 965 |
| 2',3'-Didesoxy-3'-chlor-5-methylcytidin | 0,003 | 18,5 ± 2,6 | 96 | 13450 ± 1465 |
| | 0,01 | 9,6 ± 1,2 | 50 | 14820 ± 1550 |
| | 0,03 | 7,1 ± 0,8 | 37 | 14370 ± 1620 |
| | 0,1 | 3,4 ± 0,4 | 18 | 15185 ± 1735 |
| | 0,3 | 3,2 ± 0,5 | 17 | 13895 ± 1540 |
| | 1,0 | 0,4 ± 0,1 | 2 | 12270 ± 1590 |
| | 3,0 | < 0,1 | < 1 | 10065 ± 1855 |

(Tabelle 4, Fortsetzung)

| Nucleosid | Konz. (µM) | HBsAg (ng/ml) | (%) | $^3$H-DNA (dpm/Kultur) |
|---|---|---|---|---|
| 2',3'-Didesoxy-3'-amino-5-methylcytidin | 0,003 | 13,9 ± 2,9 | 72 | 16020 ± 1790 |
| | 0,01 | 8,5 ± 1,3 | 44 | 15445 ± 1830 |
| | 0,03 | 7,0 ± 1,1 | 36 | 14180 ± 1650 |
| | 0,1 | 4,8 ± 0,6 | 25 | 14350 ± 1570 |
| | 0,3 | 3,2 ± 0,4 | 17 | 12160 ± 1860 |
| | 1,0 | 0,3 ± 0,1 | 2 | 8150 ± 1125 |
| | 3,0 | < 0,1 | < 1 | 2100 ± 430 |
| ohne | - | 17,5 ± 2,8 | 100 | 17190 ± 1980 |
| Arabinosyl-5-methylcytosin | 0,003 | 16,8 ± 1,7 | 96 | 17755 ± 1750 |
| | 0,01 | 12,4 ± 1,2 | 71 | 17990 ± 1635 |
| | 0,03 | 10,3 ± 1,2 | 59 | 19075 ± 1905 |
| | 0,1 | 3,6 ± 0,5 | 21 | 16225 ± 1825 |
| | 0,3 | 0,6 ± 0,3 | 3 | 18600 ± 1865 |
| | 1,0 | < 0,1 | < 1 | 12415 ± 1070 |
| | 3,0 | < 0,1 | < 1 | 8470 ± 935 |
| 2',3'-Didehydro-2',3'-didesoxycytidin | 0,003 | 18,7 ± 2,8 | 107 | 17940 ± 1865 |
| | 0,01 | 12,1 ± 1,6 | 69 | 18410 ± 1895 |
| | 0,03 | 5,8 ± 1,2 | 33 | 15685 ± 1740 |
| | 0,1 | 0,8 ± 0,2 | 5 | 19930 ± 1830 |
| | 0,3 | 0,4 ± 0,2 | 2 | 14270 ± 1655 |
| | 1,0 | < 0,1 | < 1 | 12350 ± 1310 |
| | 3,0 | < 0,1 | < 1 | 6535 ± 790 |

(Tabelle 4, Fortsetzung)

| Nucleosid | Konz. (µM) | HBsAg (ng/ml) | HBsAg (%) | $^3$H-DNA (dpm/Kultur) |
|---|---|---|---|---|
| 2'-3'-Didehydro-2',3'-didesoxyadenosin | 0,003 | 16,9 ± 2,6 | 96 | 19650 ± 1935 |
| | 0,01 | 12,4 ± 1,8 | 71 | 17215 ± 1760 |
| | 0,03 | 4,7 ± 0,6 | 27 | 18975 ± 1957 |
| | 0,1 | 0,5 ± 0,2 | 3 | 16230 ± 1745 |
| | 0,3 | < 0,1 | < 1 | 19400 ± 1926 |
| | 1,0 | < 0,1 | < 1 | 9930 ± 1130 |
| | 3,0 | < 0,1 | < 1 | 4265 ± 555 |
| 2',3'-Didesoxy-3'-fluor-2,6-diaminopurinribosid | 0,003 | 15,9 ± 2,7 | 91 | 19535 ± 1835 |
| | 0,01 | 3,2 ± 0,6 | 18 | 16820 ± 1775 |
| | 0,03 | 2,0 ± 0,5 | 11 | 17265 ± 1895 |
| | 0,1 | < 0,1 | < 1 | 19990 ± 1820 |
| | 0,3 | < 0,1 | < 1 | 15125 ± 1795 |
| | 1,0 | < 0,1 | < 1 | 18885 ± 1950 |
| | 3,0 | < 0,1 | < 1 | 17740 ± 1670 |
| 2',3'-Didesoxy-3'-fluor-guanosin-5'-methyl-phosphonat | 0,003 | 15,3 ± 2,8 | 87 | 18940 ± 1963 |
| | 0,01 | 4,0 ± 0,6 | 23 | 17630 ± 1740 |
| | 0,03 | 1,9 ± 0,5 | 11 | 16495 ± 1855 |
| | 0,1 | < 0,1 | < 1 | 17665 ± 1893 |
| | 0,3 | < 0,1 | < 1 | 17920 ± 1975 |
| | 1,0 | < 0,1 | < 1 | 15250 ± 1640 |
| | 3,0 | < 0,1 | < 1 | 12935 ± 1460 |

Beispiel 1

1-(2,3-Didesoxy-3-fluor-β-D-ribofuranosyl)-5-formylcytosin

1-(5-O-Acetyl-2,3-didesoxy-3-fluor-β-D-ribofuranosyl)-5-methylcytosin (0,57 g, 2 mmol) wird in 50 ml Tetrachlormethan unter Erhitzen zum Rückfluß gelöst (Photolampe, 500 W). Es werden 10 mmol Brom mit Hilfe von trockenem Stickstoff über einen Zeitraum von 6 h in die Reaktionslösung eingeleitet. Nach dem

Abkühlen werden 20 ml einer gesättigten Natriumhydrogencarbonat-Lösung hinzugefügt; die organische Phase wird abgetrennt und im Vakuum zur Trockne eingeengt. Der Rückstand wird in 30 ml Methanol gelöst und mit 1,5 mol Natriummethylat versetzt. Die Lösung wird 20 min unter Rückfluß erhitzt, nach dem Erkalten mit Essigsäure neutralisiert und schließlich im Vakuum eingeengt. Es wird ein glasartiger Rückstand erhalten, der säulenchromatographisch an Kieselgel mit Chloroform (10 % Methanol) gereinigt wird.
Massenspektrometrie: m/z 257 ($M^+$, $C_{10}H_{12}N_3O_4F$).

Beispiel 2

2′,3′-Didehydro-2′,3′didesoxy-5-fluorcytosin

Zu einer Lösung von K-tert-butylat (0,56 g, 5 mmol) in 15 ml DMF werden 0,46 g (0,2 mmol) 1-(2-Desoxy-3,5-epoxy-β-D-threo-pentofuranosyl)-5-fluorcytosin gegeben; das Gemisch wird 5 h bei Raumtemperatur gerührt. Anschließend wird zur Trockne eingeengt. Der resultierende Rückstand wird in 25 ml Wasser gelöst und mit DOWEX WX8 ($H^+$-Form) behandelt. Nach dem Filtrieren wird das Lösungsmittel im Vakuum entfernt. Der Rückstand wird in 5 ml 25%igem Methanol gelöst und an DOWEX-1 (OH-Form) säulenchromatographisch getrennt. Die Elution mit 25%igem Methanol ergibt Fraktionen, aus denen nach dem Entfernen des Lösungsmittels die Zielverbindung als ein öliger Rückstand erhalten wird. Aus kaltem Ethanol (HCl) wird das entsprechende Hydrochlorid isoliert.
Massenspektrometrie: m/z 227 ($M^+$, $C_9H_{10}N_3O_3F$).

Beispiel 3

1-β-D-Arabinofuranosyl-5-hydroxymethylcytosin

1,9 g (5 mmol) 1-(2,3,5-Tri-O-acetyl-β-D-arabinofuranosyl)-5-methylcytosin werden in 100 ml Tetrachlormethan durch Erhitzen zum Rückfluß (Photolampe, 500 W) gelöst. Mit Hilfe eines Stroms von trockenem Stickstoff werden 15 mmol Brom während eines Zeitraumes von 6 Std. in die Lösung eingeleitet. Nach dem Erkalten werden zum Reaktionsgemisch 50 ml einer gesättigten Natriumhydrogencarbonat-Lösung gegeben. Die organische Phase wird abgetrennt und zur Trockne eingeengt. Die O-Acetylgruppen werden in herkömmlicher Weise mit Methanol/Ammoniak entfernt. Der nach dem Vertreiben des Lösungsmittels verbleibende Rückstand wird an Kieselgel mit Chloroform (5% Methanol) als Elutionsmittel gereinigt. Die entsprechenden Fraktionen, die das Produkt enthalten, werden gesammelt, und das Lösungsmittel wird im Vakuum entfernt. Aus dem Rückstand wird mit kaltem Methanol die kristalline Zielverbindung erhalten.
Massenspektrometrie: m/z 257 ($M^+$, $C_{10}H_{15}N_3O_5$).

Beispiel 4

1-β-D-Arabinofuranosyl-5-methylcytosin

1-(2,3,5-Tri-O-acetyl-β-D-arabinofuranosyl)-4-thiothymin (2 g, 5 mmol) wird in einem verschlossenen Stahlcontainer mit 50 ml flüssigem Ammoniak für zwei Tage auf 50 °C erhitzt. Nach dem Verdampfen des Lösungsmittels wird der sirupöse Rückstand an Kieselgel säulenchromatographisch mit Chloroform (15% Methanol) als Elutionsmittel gereinigt. Die Titelverbindung wird als Hydrochlorid aus Methanol (HCl) erhalten,
F. 191 °C (Zers.)
Massenspektrometrie: m/z 257 ($M^+$, $C_{10}H_{15}N_3O_5$).

Beispiel 5

2',3'-Didesoxy-3',5-difluorcytidin

5'-O-Acetyl-2',3'-didesoxy-3',5-difluoruridin (0,12 mmol) wird in 3 ml Pyridin gelöst und mit 34,5 mg (0,5 mmol) 1,2,4-Triazol sowie mit 86 mg (0,35 mmol) p-Chlorphenoxyphosphorsäuredichlorid versetzt. Das Reaktionsgemisch verbleibt für eine Woche bei Raumtemperatur. Anschließend werden 15 ml Dioxan und 3 ml konzentrierte Ammoniaklösung zugesetzt. Nach 24 h wird das Lösungsmittel im Vakuum entfernt; der verbleibende braune Rückstand wird in 3 ml Wasser gelöst und an DOWEX 50 Wx8 ($H^+$-Form) getrennt. Zunächst, wird mit Wasser (420 ml), anschließend mit Ammoniaklösung (3%, 400 ml) eluiert. Die ammoniakalische Lgsung wird im Vakuum zur Trockne eingeengt, der Rückstand an Kie-selgel säulenchromatographisch gereinigt. Aus den entsprechenden Fraktionen werden 10 mg der Titelverbindung isoliert.
F. 219 °C (Methanol)
Massenspektrometrie: m/z 247 ($M^+$, $C_9H_{11}N_3O_3F_2$).

Beispiel 6

3'-Chlor-2',3'-didesoxy-5-methylcytidin

Eine Lösung von 5'-O-Acetyl-3'-chlor-3'-desoxy-thymidin (6 mmol), 1,57 g (22,7 mmol) 1,2,4-Triazol und 4,4 g (17,9 mmol) p-Chlorphenoxyphosphorsäuredichlorid in 100 ml Pyridin wird für eine Woche bei Raumtemperatur belassen. Anschließend wird ein Gemisch von 50 ml Dioxan und 20 ml konzentrierter Ammoniaklösung hinzugefügt. Nach 24 Stunden wird das Lösungsmittel entfernt und der Rückstand säulenchromatographisch zunächst an DOWEX 50 Wx8, danach an Kieselgel gereinigt. Aus den entsprechenden Fraktionen wird 3'-Chlor-2',3'-didesoxy-5-methylcytidin erhalten und nach Umwandlung in das Hydrochlorid aus Methanol umkristallisiert.
F. 203 - 205 °C (Methanol)
Massenspektrometrie: m/z 259 ($M^+$, $C_{10}H_{14}N_3O_3Cl$).

Beispiel 7

5-Brom-3'-chlor-2',3'-didesoxycytidin

Die Titelverbindung wird aus 5'-O-Acetyl-5-brom-3'-chlor-2',3'-didesoxyuridin analog der in Beispiel 6 beschriebenen Methode dargestellt und als Hydrochlorid isoliert.
Massenspektrometrie: m/z 324 ($M^+$, $C_9H_{11}N_3O_3BrCl$).

Beispiel 8

2',3'-Didehydro-2',3'-didesoxy-5-methylcytidin

5,5 g (11,3 mmol) 4-N-Benzoyl-2'-desoxy-3',5'-di-O-mesylcytidin werden in 350 ml Ethanol gelöst, mit 37,5 ml 1 n NaOH und 87 ml Wasser versetzt und 2 h unter Rückfluß erhitzt. Nach dem Erkalten wird die Lösung zur Trockne eingeengt. Der verbleibende Rückstand wird mit heißem Aceton (5 x 50 ml) extrahiert. Aus der vereinigten Acetonlösung wird das Lösungsmittel im Vakuum entfernt. Das zurückbleibende gelbe Öl wird in, 30 ml DMSO gelöst, dem 1,2 g (10,8 mmol) K-tert.-butylat hinzugefügt werden. Das Reaktionsgemisch verbleibt über Nacht bei Raumtemperatur und wird anschließend im Vakuum weitgehendst zur Trockne eingeengt. Der Rückstand wird in Wasser gelöst und mit Essigsäure auf pH 9 gebracht. Nach dem Entfernen des Lösungsmittels wird das erhaltene sirupöse Material säulenchromatographisch an Kieselgel mit Chloroform (10% Methanol) als Elutionsmittel gereinigt.
Massenspektrometrie: m/z 223 ($M^+$, $C_{10}H_{13}N_3O_3$).

Beispiel 9

9-(2,3-Didesoxy-3-fluor-β-D-ribofuranosyl)-6-thioguanin

Zu einer Suspension von 9(5-O-Benzoyl-2-desoxy-β-D-threo-pentofuranosyl)-6-thioguanin (775 mg, 2 mmol) in 100 ml 1,2-Dichlorethan wird 1 ml Diethylaminoschwefeltrifluorid gegeben; das Reaktionsgemisch verbleibt 4 h unter Rühren bei Raumtemperatur. Danach wird Natriumhydrogencarbonat (1 g) hinzugefügt, nach kurzzeitigem Rühren filtriert und schließlich das Filtrat im Vakuum zur Trockne eingeengt. Die säulenchromatographische Reinigung des Rückstandes an Kieselgel mit Chloroform (10% Methanol) als Elutionsmittel liefert 210 mg 5′-O-Benzoyl-2′,3′-didesoxy-3′-fluor-6-thioguanosin.
Massenspektrometrie m/z 389 ($M^+$, $C_{17}H_{16}N_5O_3SF$)

Die Benzoylgruppe wird üblicherweise mit Methanol/Ammoniak (gesättigt bei 0 °C) entfernt. Eine säulenchromatographische Reinigung an Kieselgel liefert 88 mg der Titelverbindung.
Massenspektrometrie: m/z 285 ($M^+$, $C_{10}H_{12}N_5O_2SF$).

Beispiel 10

9-(2,3-Didesoxy-β-D-glycero-pent-2-enofuranosyl)adenin

2′-Desoxy-3′-O-tosyl-adenosin (1 g, 2,47 mmol) wird in 5 ml DMF gelöst Dann werden 0,54 g (10 mmol) Natriummethylat in 20 ml DMF zugesetzt, die gelbe Lösung wird 45 min bei Raumtemperatur gerührt. Anschließend werden 200 ml Methanol hinzugefügt; Lösung wird mittels Amberlite IRC-50 ($H^+$-Form) neutralisiert. Nach dem Entfernen des Austauscherharzes wird das Lösungsmittel im Vakuum entfernt und der resultierende Festkörper mehrmals mit heißem Aceton extrahiert. Die vereinigten Extrakte werden auf ein Volumen von 20 ml konzentriert. Die Titelverbindung wird kristallin erhalten (0,36 g) und aus Aceton umkristallisiert.
F. 187-190 °C.

Beispiel 11

2′,3′-Didesoxy-3′-fluor-5-methylcytidin-5′-hydrogenphosphonat

Imidazol (360 mg, 5,3 mmol) wird in 15 ml Acetonitril gelöst und im Eisbad auf 0 °C gekühlt. Zur Lösung werden Phosphortrichlorid (0,15 ml, 1,6 mmol) und Triethylamin (0,78 ml, 5,6 mmol) gegeben, und es wird für 15 min gerührt. Anschließend werden 0,48 g (2 mmol) 2′,3′-Didesoxy-3′-fluor-5-methylcytidin, gelöst in 25 ml Acetonitril, hinzugefügt; das Reaktionsgemisch verbleibt unter ständigem Rühren 5 h bei Raumtemperatur. Nach der Zugabe von 25 ml Wasser wird die Lösung im Vakuum eingeengt und mehrmals mit Pyridin/Triethylamin (3/1) codestilliert. Der schließlich erhaltene Rückstand wird säulenchromatographisch an Kieselgel mit Chloroform (10% Methanol) gereinigt.

Beispiel 12

Injektionslösung

Aus 2′,3′-Didesoxy-3′-fluor-5-methylcytidin und physiologischer Kochsalzlösung bereitet man die erforderliche Menge einer 3%-igen Lösung.

Beispiel 13

Tabletten und Dragees

Pulverisiertes 2′,3′-Didesoxy-3′-fluor-5-methylcytidin wird mit einem oder mehreren üblichen Trägerstoffen, wie Stärke, Talk, Magnesiumstearat, Kaliumstearat, Stearinsäure, Paraffin, Cetylalkohol, Pectin, Saccharose, Gummi arabicum, Dextrin, zu Tabletten oder Dragees verarbeitet.

## Ansprüche

1. Pyrimidinnucleoside und Purinnucleoside der allgemeinen Formeln I, II, III, IV und V,

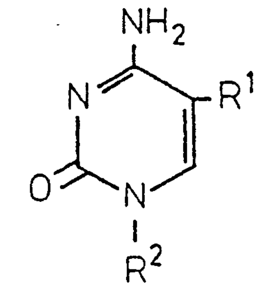

(I),

worin bedeuten:
$R^1$ CHO, $NH_2$, OH, SH
und
$R^2$

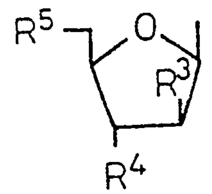

mit $R^3$ = H, OH
$R^4$ = H, F, Cl, $NH_2$, $N_3$
$R^5$ - OH, O-Acetyl, O-Palmitoyl, O-Alkoxycarbonyl, Phosphonat, Mono-, Di-, Triphosphat bzw. andere Precursorgruppen für die 5-Hydroxygruppe,

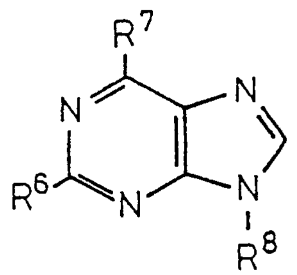

(II),

worin bedeuten:
$R^6$ H, OH, F, Cl, Br, $NH_2$, SH, N-Dialkyl, insbesondere bis $C_3$,
$R^7$ H, OH, F, Cl, Br, $NH_2$, SH, N-Dialkyl, insbesondere bis $C_3$,
$R^8$ 2,3-Didesoxy-3-aminoribofuranosyl, 2,3-Didesoxy-3-chlorribofuranosyl, 2,3-Didesoxy-3-azidoribofuranosyl, 2,3-Didesoxy-3-fluorribofuranosyl, Arabinofuranosyl 3-Fluorarabinofuranosyl, 2,3-Didehydro-2,3-didesoxyribofuranosyl bzw. deren 5-Phosphonat 5-O-Acetyl-, 5-O-Palmitoyl-, 5-O-alkoxycarbonyl-Derivate oder deren 5-Mono-, 5-Di-, oder 5-Triphosphate (als freie Säuren oder Alkali-, Ammonium- oder Alkylammonium salze) bzw. andere Precursorgruppen für die 5-Hydroxygruppe, mit folgenden Einschränkungen:
Wenn $R^6$ und $R^7$ = H, OH, $NH_2$,
dann ist
$R^8$ ≠ 2,3-Didesoxy-3-chlorribofuranosyl, 2,3-Didesoxy-3-aminoribofuranosyl, 2,3-Didesoxy-3-azidoribofuranosyl, 2,3-Didesoxy-3-fluorribofuranosyl, Arabinofuranosyl 3-Fluorarabinofuranosyl, 2,3-Didehydro-2,3-didesoxyribofuranosyl;
oder wenn $R^6$ = F und $R^7$ = $NH_2$ bzw. $R^6$ = $NH_2$ und $R^7$ = SH, dann ist $R^8 \neq$ 3-Fluorarabinofuranosyl bzw. 2,3-Didesoxy-3-fluorribofuranosyl,
oder wenn $R^6$ = Cl und $R^7$ = $NH_2$, dann ist $R^8 \neq$ 2,3-Didehydro-2,3-didesoxyribofuranosyl;

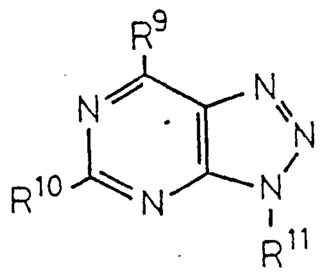

(III),

worin bedeuten:
$R^9$ und $R^{10}$ H, OH, $NH_2$ und
$R^{11}$ 2,3-Didehydro-2,3-didesoxyribofuranosyl oder

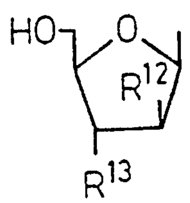

mit $R^{12}$ = H, OH
$R^{13}$ = H F, Cl, $NH_2$, $N_3$
bzw. mit einer Precursorgruppe für die 5-Hydroxygruppe wie bei $R^8$;

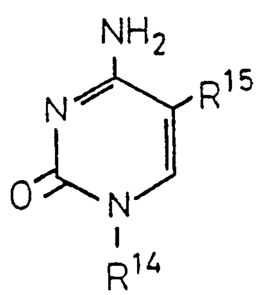

(IV),

worin bedeuten:
$R^{14}$ 2,3-Didehydro-2,3-didesoxyribofuranosyl bzw. eine Precursorgruppe für die 5-Hydroxygruppe wie bei $R^8$ und
$R^{15}$ F, Br, J, $CH_3$, $CH_2OH$, $CH_2CH_3$, CHO;

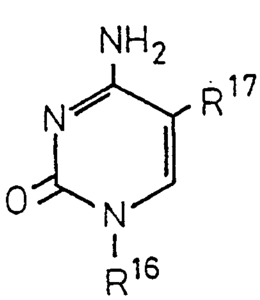

(V),

worin bedeuten:
$R^{16}$ Arabinofuranosyl, 2,3-Didesoxy-3-aminoribofuranosyl bzw. mit einer Precursorgruppe für die 5-Hydroxygruppe wie bei $R^8$, und
$R^{17}$ Cl, Br, $CH_2OH$, $CH_2CH_3$, $CH=CH_2$, $CH=CHBr$.

2. 2',3'-Didesoxy-3'-fluor-5-formylcytidin der Formel I.

3. 2',3,'-Didesoxy-3'-fluorribofuranosyl-6-chlorguanin der Formel II.

4. 2',3'-Didesoxy-3'-fluorribofuranosyl-8-azaguanin der Formel III.

5. 2',3'-Didehydro-2',3'-didesoxy-5-methylcytidin der Formel IV.

6. Arabinofuranosyl-5-hydroxymethylcytosin der Formel V.

7. Verfahren zur Herstellung der Verbindungen nach einem der Ansprüche 1 bis 6, dadurch gekennzeichnet, daß man
- zur Herstellung der Verbindungen der Formel I entweder 3'-modifizierte 5-Methylpyrimidinnucleoside zunächst bromiert und anschließend das exocyclische Brom eliminiert, oder das entsprechende 5-Bromderivat substituiert;
- zur Herstellung der Verbindungen der Formeln II und III die entsprechenden Purine in einer Transglycosidierungsreaktion mit dem entsprechend 3'-modifizierten Zucker versieht oder das entsprechende 3'-C-Tosylderivat einer Eliminierungsreaktion unterzieht;
- zur Herstellung der Verbindungen der Formel IV die 3',5'-Di-O-mesylverbindungen, die am $N^4$ geschützt sind, einer Eliminierungsreaktion unterzieht
bzw.
- zur Herstellung der Verbindungen der Formel V das entsprechende Arabinofuranosylcytosin bzw. 2',3'-Didesoxy-3'-aminocytidin in 5-Position durch Halogen substituiert oder das 5-Ethylarabinofuranosylcytosin bzw. 2',3'-Didesoxy-3'-amino-5-ethylcytidin durch Bromierung und nachfolgende Eliminierung abwandelt.

8. Pharmazeutische Mittel, insbesondere zur Prophylaxe und/oder Behandlung von durch Viren, insbesondere Hepatitis B-Viren, verursachten Infektionen, gekennzeichnet durch mindestens eine Verbindung der allgemeinen Formeln I und/oder II und/oder III und/oder IV und/oder V nach einem der Ansprüche 1 bis 6 als Wirkstoffe neben üblichen galenischen Hilfs-, Träger-und/oder Verdünnungsmitteln.

9. Verwendung der Verbindungen der allgemeinen Formeln I bis V nach einem der Ansprüche 1 bis 6 und von Verbindungen der allgemeinen Formeln VI und VII,

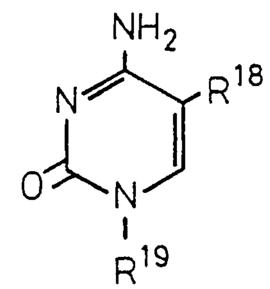

(VI),

in der bedeuten:
$R^{18}$ F, Cl, Br, J, $CH_3$, $CH_2OH$, $C_2H_5$ und
$R^{19}$ 2,3-Didesoxyribofuranosyl, 2,3-Didesoxy-3-azidoribofuranosyl, 2,3-Didesoxy-3-fluorribofuranosyl, 2,3-Didesoxy-3-chlorribofuranosyl, 3-Fluorarabinofuranosyl bzw. eine Precursorgruppe für die 5 Hydroxygruppe wie bei $R^8$ in Anspruch 1
oder
$R^{18}$ F, J, $CH^3$ und
$R^{19}$ Arabinofuranosyl, 2,3-Didesoxy-3-aminofuranosyl bzw. eine Precursorgruppe für die 5 Hydroxygruppe wie bei $R^8$ in Anspruch 1
oder
$R^{18}$ H, Cl und
$R^{19}$ 2,3-Didehydro-2,3-didesoxyribofuranosyl bzw. eine Precursorgruppe für die 5 Hydroxygruppe wie bei $R^8$ in Anspruch 1

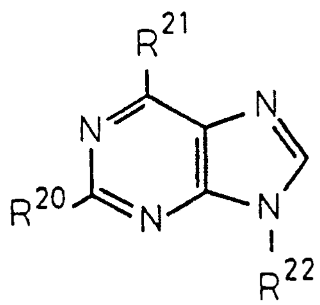

(VII),

in der bedeuten:
$R^{20}$ $NH_2$ und $R^{21}$ SH,
$R^{20}$ $NH_2$ und $R^{21}$ H
$R^{20}$ $NH_2$ und $R^{21}$ $NH_2$
$R^{20}$ F und $R^{21}$ $NH_2$, und
$R^{22}$ 2,3-Didesoxy-3-fluorribofuranosyl, 3-Fluorarabinofuranosyl bzw. eine Precursorgruppe für die 5 Hydroxygruppe wie bei $R^8$ in Anspruch 1
oder
$R^{20}$ H und $R^{21}$ $NH_2$
$R^{20}$ $NH_2$ und $R^{21}$ OH
$R^{20}$ $NH_2$ und $R^{21}$ $NH_2$, und
$R^{22}$ 2,3-Didehydro-2,3-didesoxyribofuranosyl 2,3-Didesoxy-3-aminoribofuranosyl bzw. eine Precursorgruppe für die 5 Hydroxygruppe wie bei $R^8$ in Anspruch 1
oder
$R^{20}$ CH und $R^{21}$ $NH_2$, und
$R^{22}$ 2,3-Didesoxy-3-fluorribofuranosyl bzw. eine Precursorgruppe für die 5 Hydroxygruppe wie bei $R^8$ in Anspruch 1,
zur Herstellung pharmazeutischer Mittel, insbesondere zur Prophylaxe und/oder Behandlung von durch Hepatitis B-Viren verursachten Infektionen, insbesondere in der Humanmedizin.

10. Verwendung von Arabinofuranosyl-5-methylcytosin, 2′,3′-Didesoxy-3′-fluor-5-methylcytidin-5′-hydrogenphosphonat, 2′,3′-Didesoxy-3′-fluor-5-methylcytidin, 2′,3′-Didesoxy-3′-azido-5-methylcytidin, 2′,3′-Didesoxy-1′-aminoribofuranosylguanin, 2′,3′-Didehydro-2′,3′-didesoxyribofuranosylguanin, 2′,3′-Didesoxy-3′-fluorarabinofuranosyl-5-methylcytosin und/oder 2′,3′-Didesoxy-3′-fluorguanosinphosphonat gemäß Anspruch 9.